# EUROPEAN PATENT SPECIFICATION

(11) **EP 0 910 383 B1**
(45) Date of publication and mention of the grant of the patent: **15.10.2003**
(21) Application number: 97930938.2
(22) Date of filing: 23.06.1997
(51) Int. Cl.: A61K 31/66

(54) **THE USE OF GROWTH FACTOR MODULATING COMPOUNDS**
VERWENDUNG VON MITTELN DIE DEN WACHSTUMSFAKTOR BEEINFLUSSEN
UTILISATION DE COMPOSES MODULATEURS DU FACTEUR DE CROISSANCE

(30) Priority: 24.06.1996 SE 9602463
(43) Date of publication of application: 28.04.1999
(60) Divisional application: 02003260.3
(73) Proprietor: Bioneris AB,, 181 31 Lidingö (SE)
(72) Inventor: SIREN, Matti, Helsingfors 17, Finland (FI)
(74) Representative: HOFFMANN - EITLE
(86) International application number: SE9701115
(87) International publication number: WO97049408

(56) References cited:
- EP-A- 0 252 227
- EP-A- 0 269 105
- EP-A- 0 359 256
- EP-A- 0 420 428
- WO-A-91/09601
- WO-A-94/09790
- WO-A-95/14475
- WO-A-95/14476
- US-A- 4 952 396
- CIRKULATION RESEARCH, Volume 75, No. 5, November 1994, S.A. RODT et al., "The Anti-Inflammatory Agent Alpha-Trinositol Exerts Its Edema-Preventing Effect Through Modulation of Betal Integrin Function", pages 942-948.

## Description

Growth factors comprise a family of polypeptides with a manyfold of properties regulating for example cell proliferation and cell metabolism. As being multifunctional molecules, they may stimulate or inhibit cell proliferation as well as affect cell function depending on the type of the target cells and the presence of other signal peptides. The family of polypeptides include for example platelet derived growth factor (PDGF), epidermal growth factor (EGF), transforming growth factors (TGF-, TGF-β), insulin like growth factors (IGF-1, IGF-2), fibroblast growth factors (a FGF, b FGF), vascular endothelial growth factor (VEGF), nerve growth factor (NGF) and bone morphogenetic proteins (BMP).

The activities of these growth factors are considered to be casual components of several conditions such as cardiovascular conditions, for example diabetes, inflammatory conditions for example rheumatoid arthritis, analgetic conditions, viral conditions, carcinogenic conditions, several eye diseases and wound healing.

Angiogenesis is under normal conditions under stringent control but in many pathological conditions e.g. solid tumours, rheumatoid arthritis, diabetic retinopathy and artherosclerosis unregulated vessel proliferation occurs. The angiogenetic process is controlled by a balance between many positive and negative regulating signals where growth factors such as TGF, FGF and VEGF play a dominant role.

Under normal conditions in the body growth factor activity is to a large extent regulated by the interactions with heparin and heparan sulfate. The interaction between these polyanionic glycosaminoglycans and growth factors is thought to be of functional significance serving as storage depots for growth factors and protecting them from various degradative processes (Vlodavsky, I., Fuks, Z., Ishai-Michaeli, R., Bashkin, P., Levi, E., Korner, G., Bar-Shavit, R., and Klagsbrun, M. (1991) J. Cell. Biochem. 45, 167-176).

Under some circumstances the administration of growth factors as therapeutic agents has been utilized. However the limited stability of this type of molecules reduces their activity before reaching the target in the body.

Under abnormal conditions when growth factor regulation is required to a larger extent than in the normal situation, the administration of heparin and derivatives of heparin has been considered. However the administration of these type of compounds renders side-effects like anticoagulant activity which limits their possible usage.

Other sulphonated compounds like suramin, a polysulphonated napthylurea, has been shown to have activity in the treatment of adrenocortical carbinoma but the limitation also for this type of compounds is the narrow margin between the dose required to achieve anti-tumor activity and the dose leading to the onset of toxic side effects.

According to the present invention the use of a compound containing a high density, negatively charged domain of vicinally oriented radicals for the preparing of a medicament providing a growth factor modulating activity in mammals including man is described.

WO 95/14476 for example discloses the use of IP₃(D-myo-inositol-1,2,6-triphosphate) in the treatment of inflammatory conditions and conditions of angiogenesis.

In the invention, the negatively charged domain comprises at least three vicinal phosphorus-containing radicals.

The invention relates to the use of a compound wherein the phosphorus-containing radicals have the following formula: 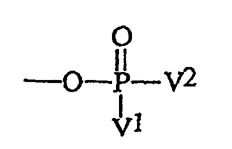 wherein V¹ and V² are the same or different and are OH, (CH₂)ₚOH, COOH, CONH₂, CONOH,
(CH₂)ₚCOOH, (CH₂)ₚCONH₂, (CH₂)ₚCONOH, (CH₂)ₚSO₃H,
(CH₂)ₚSO₃NH₂, (CH₂)ₚNO₂, (CH₂)ₚPO₃H₂, O(CH₂)ₚOH,
O(CH₂)ₚCOOH, O(CH₂)ₚCONH₂, O(CH₂)ₚCONOH, O(CH₂)ₚSO₃H,
O(CH₂)ₚSO₃NH₂, O(CH₂)ₚNO₂, O(CH₂)ₚPO₃H₂ or CF₂COOH, and
p is 1 to 4.

In the invention, the phosphorus-containing radicals are phosphonates or phospates or derivatives thereof.

In the invention, the backbone to the high density negatively charged region of vicinally oriented radicals is selected from a specific group of monosaccharides.

In one preferred embodiment of the invention the compounds are phosphates, phosphonates or phosphinates of cyclohexane such as 1, 2, 3β-cyclohexane-1,2,3-trioltrisphosphate.

The monosacharide is selected from the group of D/L-ribose, D/L-arabinose, D/L-xylose, D/L-lyxose, D/L-allose, D/L-altrose, D/L-glucose, D/L-mannose, D/L-gulose, D/L-idose, D/L-galactose, D/L-talose, D/L-ribulose, D/L-xylulose, D/L-psicose, D/L-sorbose, D/L-tagatose and D/L-fructose . In preferred embodiments of this type of the invention the compounds are phosphates, phosphonates or phosphinates of these sacharides. The number of phosphate-, phosphonate- or phosphinate radicals per sacharide unit is at least three. The remaining hydroxyl groups on the sacharide moiety may be derivatized in the form of ethers or esters. In many instances the ether form is desired as this type of radical propongs the stability and half-life in vivo as the susceptibility to enzymatic degradation is reduced. In one preferred embodiment of this type of the invention the compound is selected from the group of mannose-2,3,4-trisphosphate, galactose-2,3,4-trisphosphate, fructose-2,3,4-trisphosphate, altrose-2,3,4-trisphosphate and rhamnose-2,3,4-trisphosphate. In one most preferred embodiment the compound is selected from the group of R₁-6-O-R₂-α-D-mannopyranoside-2,3,4-trisphosphate, R₁-6-O-R₂-α-D-galactopyranoside-2,3,4-trisphosphate, R₁-6-O-R₂-α-D-altropyranoside-2,3,4-trisphosphate and R₁-6-O-R₂-β-D-fructopyranoside-2,3,4-trisphosphate where R₁ and R₂ independently are methyl, ethyl, propyl, butyl, pentyl or hexyl. Most preferred compounds in this type of the invention are mathyl-6-O-butyl-α-D-mannopyranoside-2,3,4-trisphosphate, methyl-6-O-butyl-α-D-galactopyranoside-2,3,4-trisphosphate, methyl-6-O-butyl-α-D-glycopyranoside-2,3,4-trisphosphate, methyl-6-O-butyl-α-D-altropyranoside-2,3,4-trisphosphate, methyl-6-O-butyl-β-D-fructopyranoside-2,3,4-trisphosphate, 1,5-anhydro-D-arabinitol-2,3,4-trisphosphate, 1,5-anhydraxylitol-2,3,4-trisphosphate, 1,2-O-ethylene-β-D-fructopyranoside-2,3,4-trisphosphate, methyl-α-D-rhamnopyranoside-2,3,4-- trisphosphate, methyl-α-D-mannopyranoside-2,3,4-triphosphate, methyl-6-O-butyl-α-D-mannopyranoside-2,3,4-tris-(carboxymethylphosphate), methyl-6-O-butyl-α-D-mannopyranoside-2,3,4-tris-(carboxymethylphosphonate), methyl-6-O-butyl-α-D-mannopyranoside-2,3,4-tris(hydroxymethylphosphonate), methyl-6-O-butyl-α-D-galactopyranoside-2,3,4-tris(carboxymethylphosphate), methyl-6-O-butyl-α-D-galactopyranoside-2,3,4-tris(carboxymethylphosphonate), methyl-6-O-butyl-α-D-galactopyranoside-2,3,4-tris(hydroxymethylphosphonate), methyl-6-O-butyl-α-D-glucopyranoside-2,3,4-tris(carboxymethylphosphate), methyl-6-O-butyl-α-D-glucopyranoside-2,3,4-tris(carboxymethylphosphonate), methyl-6-O-butyl-α-D-glucopyranoside-2,3,4-tris(hydroxymethylphosphonate), methyl-6-O-butyl-α-D-altropyranoside-2,3,4-tris-(carboxymethylphosphate), methyl-6-O-butyl-α-D-altropyranoside-2,3,4-tris-(carboxymethylphosphonate), methyl-6-O-butyl-α-D-altropyranoside-2,3,4-tris-(hydroxymethylphosphonate), methyl-6-O-butyl-β-D-fructopyranoside-2,3,4-tris-(carboxymethylphosphate), methyl-6-O-butyl-β-D-fructopyranoside-2,3,4-tris-(carboxymethylphosphonate), methyl-6-O-butyl-β-D-fructopyranoside-2,3,4-tris-(hydroxymethylphosphonate).

In other preferred embodiments of the invention the compounds are phosphates, phosphonates or phosphinates of heterocyclic moieties such as 1,5-dideoxy-1,5-iminoarabinitol-2,3,4-trisphosphate, 1,5-dideoxy-1,5-iminoarabinitol-2,3,4-tris(carboxymethylphosphate), 1,5-dideoxy-1,5-iminoarabinitol-2,3,4-tris(carboxymethylphosphonate), 1,5-dideoxy-1,5-iminoarabinitol-2,3,4-tris(hydroxymethylphosphonate), 1,5-dideoxy-1,5-imino-N-(2-phenylethyl)arabinitol-2,3,4-trisphosphate, 1,5-dideoxy-1,5-imino-N-(2-phenylethyl)arabinitol-2,3,4-tris(carboxymethylphosphate), 1,5-dideoxy-1,5-imino-N-(2-phenylethyl)arabinitol-2,3,4-tris(carboxymethylphosphonate), 1,5-dideoxy-1,5-imino-N-(2-phenylethyl)arabinitol-2,3,4-tris(hydroxymethylphosphonate).

The growth factor modulating activity of the described compounds are expressed on at least four levels. One level is a type of interaction with growth factors such as heparin-binding growth factors and/or the specific receptors of these growth factors. The compounds according to the invention interact with domains comprising high basicity on the growth factors. This type of interaction is considered to be an important factor in connection with growth factors such as basic fibroblast growth factor, acidic fibroblast growth factor, platelet-derived growth factor, vascular endothelial growth factor and hepatocyte growth factor.

The interactions are considered to be especially important in connection with growth factors from the fibroblast growth family such as different types of acidic and basic fibroblast growth factors. Processes which are regulated by the interaction are e.g. dimerization and interactions with receptors and ligands.

Another level of interaction is a more specific interaction with the internalisation process of a group of growth factors. Many growth factors are exerting their effect after internalisation to the intracellular domain. The pathway includes an interaction/binding of the growth factor to the external part of a transmembrane receptor. Binding is followed by endocytosis i.e. the formation of a vesicle which is transferred over the plasma membrane and released into the internal part of the cell. This process of internalisation is controlled by specific assembly proteins. One specific assembly protein, AP-2 is considered to be important in the internalisation process of this group of growth factors. Within the scope of the invention it is considered that the described compounds interact with the external receptor and an ion-channel connected to AP-2. The binding of this group of growth factors to their receptor is effected by interaction with receptor bound small monophosphorylated sugars such as mannose-phosphate. When the binding to the monophosphorylated sugar is reversed by the existence of compounds according to the invention, the binding of the growth factor is retarded or does not happen at all.

The other interaction on this level is the binding of the compounds according to the invention to an ion-channel, most often a potassium-channel belonging to AP-2. Such a binding retards or in some case inhibit the internalisation process and thus retards or inhibits the consequences of growth factors entering the internal part of the cell and the subsequent metabolic events. To the group of growth factors functioning according to the described pathway belong for example epidermal growth factor, transforming growth factor, insulin growth factor and nerve growth factor.

A third level of interaction includes a very specific interaction between the compounds according to the invention and a specific group of growth factors. The negatively charged domain of the compounds according to the invention interacts with domains in the growth factor, characterized by the existence of basic amino acids such as arginine, lysine and histidine structurally arranged in a way that renders condensed binding sites on a small area. The binding area is typically not exceeding 500 square Angstrom and requires a specific stereochemistry of the binding compound. Accordingly the preferred compounds have two equatorial and one axial equatorial negatively charged groups attached to a cyclic moiety. Within this group of growth factors transforming growth factor β is mentioned as one example. The interaction is for example expressed by three lysine moieties, lysine 25, lysine 31 and lysine 37 and three phosphate radicals in a way that the distance between the radicals is less than 10 Angstrom.

The interaction between the growth factors and the compounds according to the invention is characterized by a binding constant, K_{D}. Typically, the K_{D}-value is less than 100 µM. Preferably the binding constant is less than 60 µM and most preferably less than 30 µM.

The interaction is also described as a consequence of the acid constants, pK_{A1} of the compounds according to the invention. Typically pK_{A1} is in the range of 7 to 9 and for some compounds in the range of 7 to 12.

A fourth level of interaction is expressed by a receptor interaction on the cell surface which transfer the signal from for example a specific growth factor via a signalling cascade to the internal compartments of the cell. The compounds bind to the receptor on the cell surface characterized by a IC₅₀-value less than 50 µM and preferably less than 5 µM. The binding is depending on the concentration, either allosteric or hyperbolic.

The effects described renders the growth factor modulating activity which is characteristic to the use of the compounds according to the invention.

According to the invention the compounds are most often present in a salt form or in a form where only a few of the negative charges are protonated. The salt can contain one or more cations in different combinations. Examples of cations are sodium and potassium ions.

The pharmaceutical composition according to the invention may be adminstered orally, topically, parentally, rectally or by inhalation spray in dosage forms or formulations comprising conventional, non-toxic pharmaceutically acceptable carriers, adjuvants and vehicles

The pharmaceutical composition for oral use can be present in different forms such as capsules, granules, tablets, troches, lozenges, aqueous suspensions, dispensible powders, emulsions, syrups or elixirs. When the composition is present in liquid form capsules are preferably utilized. At the use of granules, these preferably have a size of 0.15-2 mm. Either the granules can consist of the pharmaceutical composition per se or of the composition and suitable fillers. When the pharmaceutical composition is used in a tablet form, the tablets can have a weight of 50-1500 mg, preferably 50-800 mg and most preferably 100-500 mg.

Formulations for oral use include tablets which contain the active ingredient in a mixture with non-toxic pharmaceutically acceptable excipients. These excipients may be, for example, inert diluents, such as calcium carbonate, sodium chloride, lactose, calcium phosphate or sodium phosphate; granulating and disintegrating agents, for example, potato starch, or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the activ ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin, or olive oil.

For the parentaral application of the composition of this invention, typical dosage forms include intravenous, intramuscular, intraperitoneal formulations

For the rectal application of the composition of this invention, typical dosage forms include suppositories, rectal gelatin capsules (solutions and suspensions), and enemas or micro-enemas (solutions and suspensions). Thus, in a typical suppository formulation, any one of the compounds of this invention is combined with any pharmaceutically acceptable suppository base such as cocoa butter, esterified fatty acids, glycerinated gelatin, and various water-soluble or dispersible bases like polyethylene glycols and polyoxyethylene sorbitan fatty acid ester Various additives like salicylates or surfactant materials may be incorporated.

For topical use, creams, ointments, gels, solutions or the like containing the compostions are employed according to methods recognized in the art.

Naturally, the therapeutic dosage range for the compounds of the present invention will vary with the size and needs of the patient and the particular condition or disease symptom being treated.

The administration of the pharmaceutical composition according to the invention can be in a combined dosage form or in separate dosage forms.

For administration to human patients appropriate dosages can routinely be determined by those skilled in this art by extension of the results obtained in animals at various dosages. The preferred dosage for humans falls within the range of 0.1 to 100 mg compound per day and kg bodyweight, especially 0.1 to 50 mg/day/kg bodyweight.

The administration of the compounds according to the invention to mammals are considered to be benefical within the following conditions:
* Inflammatory conditions such as rheumatoid arthritis
* Tumour conditions such as glioma, prostate cancer and other conditions where inhibition of angiogenesis is crucial

According to the present invention a process of modulating growth factor activity by using a compound containing a high density, negatively charged domain of vicinally oriented radicals is described.

In the invention, the negatively charged domain comprises at least three vicinal phosphorus-containing radicals.

The invention will be further explained with the following embodiment examples however without limiting it thereto.

Examples 1 and 2 illustrate the growth factor modulating effect of 1,2-O-ethylene-β-D-fructopyranoside 2,3,4-trisphosphate (PP 35-405) and 1,5-dideoxy-1,5-imino-N-(2-phenylethyl)arabinitol-2,3,4-trisphosphate (PP 35-508) respectively, in inflammatory conditions.

Example 3 illustrates the binding of compounds accordings to the invention to spermine, while example 4 shows the analgetic effect of methyl-α-D-rhamnopyranoside-2,3,4-trisphosphate (PP 35-406). Reference Example 1 describes the interaction between IP₃ and different fibroblast growth factors in computer modelling experiments.

### Example 1

Growth factors are implicated to participate in inflammatory conditions. Carrageenan (0.1 ml, 1 %) was injected intraplantarly in one hind paw of two groups of mice, five animals in each group.

An intravenous injection of 64 mg/kg of 1,2-O-ethylene-β-D-fructopyranoside-2,3,4-trisphosphate (PP3 5-405) was administered immediately before the injection of carrageenan.

Injection of carrageenan induces inflammation which is measured by comparing the increase in paw volume using a plethysmometer.

After 3.5 hrs a comparison shows that the inflammation was retarded by 51 % in the group recieving PP35-405. Thus the administration of PP35-405 significantly counteract the inflammatory conditions.

### Example 2

In a procedure similar to the one described in Example 1, an intravenous injection of 1,5-dideoxy-1,5-imino-N-(2-phenylethyl)arabinitol-2,3,4-trisphosphate (PP35-508) was administered.

3,5 hrs after the injection of carrageenan, the inflammation was retarded with 27 % in the group recieving PP35-508. Thus the administration of PP35-508 markedly retard the inflammatory condition.

### Example 3

The interaction between a polyamine, spermine and different triphosphorylated compounds were investigated in order to determin the binding constants. Spermine is a basic polyamine comprising domains of similar basicity to basic domains in growth factors. Spermine and the different phosphorylated compounds forms different complexes and the 1:1:5 complex was used as the complex for determination of binding constants.

The following binding constants, log Kₛ was determined in 0.1 M N-tetramethylbromide at 25°C:

| Compound | log Kₛ |
|---|---|
| D-myo-inositol-1,2,6-trisphosphate | 5.16 (reference) |
| D-3,4,5-tri-O-hexanoyl-myo-inositol-1,2,6-trisphosphate, PP10-202 | 4.20 (reference) |
| D-3,4,5-tri-O-phenylcarbamoyl-myo-inositol-1,2,6-trisphosphate, PP11-201 | 4.71 (reference) |
| Methyl-6-O-butyl-α-D-mannopyranoside 2,3,4-trisphosphate, PP35-402 | 5.36 |
| Trimethylolpropane trisphosphate, PP50-202 | <1 (reference) |

The results show that the cyclic moieties have binding constants, log Kₛ, higher than 4 indicating a strong binding, while the linear compound PP50-202 does not bind.

### Example 4

Two groups of mice, 10 animals per group, were used in order to investigate the analgetic effect of methyl α-D-rhamnopyranoside-2,3,4-trisphosphate (PP 35-406). The control group was given an intravenous dose of saline while the other group was given a dose of 64 mg/kg of the sodium salt of PP 35-406. Three minutes after intravenous dosing, each rat received an intraperitoneal injection of 1 ml of a 1 % (w/w) solution of acetic acid. Directly after that procedure each animal was placed into individual observation chambers and the numbers of writhes elicited during the subsequent 10-minute period were recorded. After the observation period the animals were killed by cervical dislocation. The number of writhes during the observation period is an expression of the pain experienced by the animal. Percent protection was calculated as follows: 10x ((number of writhes in control group - number of writhes in treated animals)/(number of writhes in control group).

The protection determined for PP 35-406 was 19 % demonstrating a reduction in pain when the compound is administered.

### Ref. Example 1

The interaction between D-myo-inositol-1,2,6-trisphosphate (IP₃) and several growth factors has been examined by the utilization of computer modelling programs.

Especially the interaction between IP₃ and the fibroblast growth factor family reveals the pattern that the two axial and one equatorial phosphate group interacts with regions consisting of basic amino acids such as lysine, arginine and histidine. These domain are essential for the dimerization of growth factors but also for the interaction between growth factors and their cellular receptors.

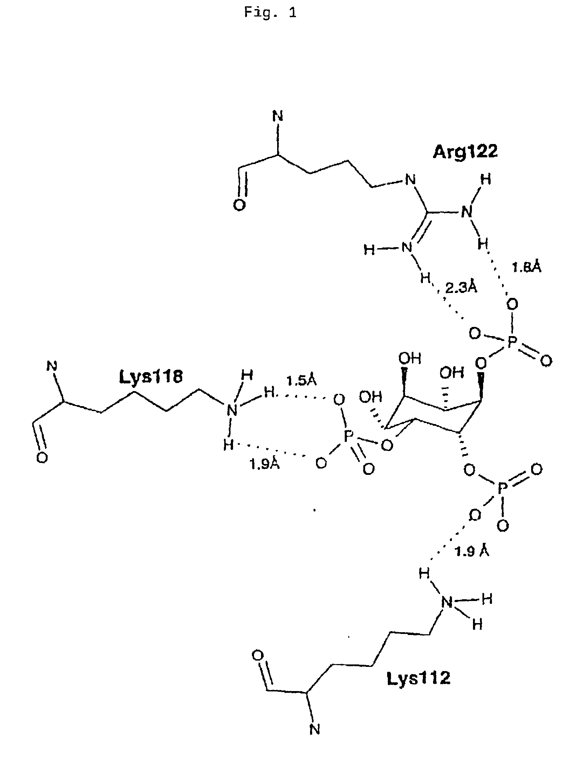

In the interaction between IP₃ and acid Fibroblast Growth Factor (a FGF) specific binding is observed with lysine 112, lysine 118 and arginine 122 in such a manner that the distance is approximately 2 Å indicating strong and firm interactions. The interaction is shown in figure 1.

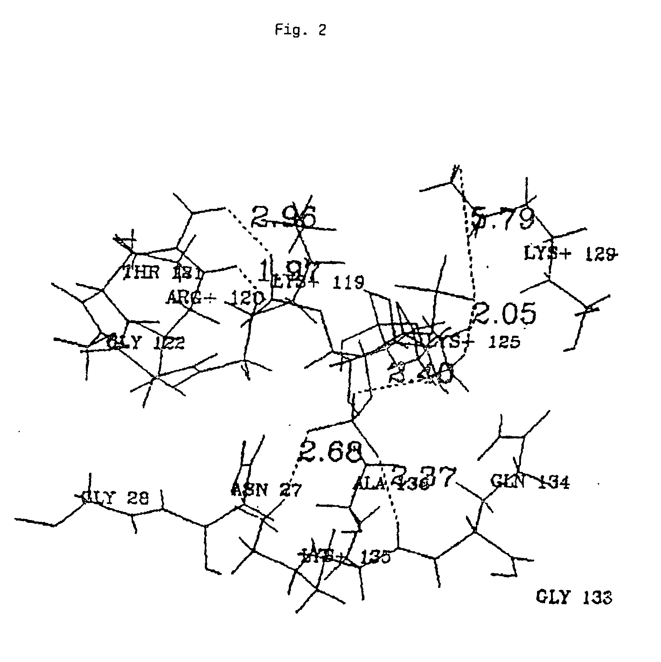

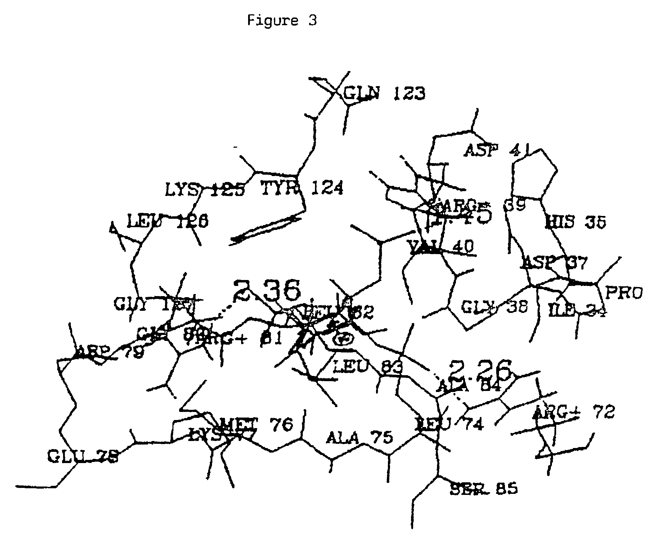

Furthermore IP₃ interacts in a very specific way with basic Fibroblast Growth Factor (b FGF). The different phosphate groups of IP₃ interacts with arginine 120; distance 1.94 Å; with lysine 125; distance 2,40 Å, with lysine 135; distance 2.37 Å and with aspargine 27; distance 2,68 Å. The interaction is further described in figure 2. A specific interaction is observed to the site in b FGF where the dimerization process is initiated. To this site IP₃ interacts with arginine 72; distance 2,26 Å, with arginine 81; distance 2.36 Å and arginine 39; distance 1.45 Å. This interaction is further described in figure 3.

One of the essential elements of the interaction is the fact that the three phosphate groups of IP₃ stereochemically is in a form where two moities are axial and one equatorial As a consequence IP₃ can be regarded as a model substance where the same type of interactions can be foreseen with other stereochemically similar compounds according to the invention.

## Claims

1. The use of a monosaccharide selected from the group of D/L-ribose, D/L-arabinose, D/L-xylose, D/L-lyxose, D/L-allose, D/L-altrose, D/L-glucose, D/L-mannose, D/L-gulose, D/L-idose, D/L-galactose, D/L-talose, D/L-ribulose, D/L-xylulose, D/L-psicose, D/L-sorbose, D/L-tagatose, and D/L-fructose, in which said monosaccharide is substituted with at least three vicinal phosphorus-containing radicals of the formula: 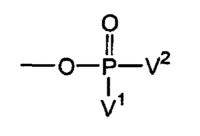 wherein
V¹ and V² are OH, (CH₂)ₚOH, COOH, CONH₂, CONOH, (CH₂)ₚCOOH, (CH₂)ₚCONH₂, (CH₂)ₚCONOH, (CH₂)ₚSO₃H, (CH₂)ₚSO₃NH₂, (CH₂)ₚNO₂, (CH₂)ₚPO₃H₂, O(CH₂)ₚOH, O(CH₂)ₚCOOH, O(CH₂)ₚCONH₂, O(CH₂)ₚCONOH, O(CH₂)ₚSO₃H, O(CH₂)ₚSO₃NH₂, O(CH₂)ₚNO₂, O(CH₂)ₚPO₃H₂ or CF₂COOH; and
p is 1 to 4,
for the preparation of a medicament for preventing, alleviating or combating inflammatory conditions in mammals including man, or conditions of angiogenesis in mammals including man wherein the angiogenesis is related to tumor conditions.

2. The use according to claim 1 wherein the tumor condition is glioma or prostate cancer.

3. The use according to claim 1 wherein the inflammatory condition is rheumatoid arthritis.

4. The use according to any of claims 1 to 3 wherein the phosphorus-containing radicals are phosphate groups.

5. The use according to any of claims 1 to 3 wherein the monosaccharide is selected from the group of mannose-2,3,4-trisphosphate, rhamnose-2,3,4-trisphosphate, galactose-2,3,4-trisphosphate, methyl-6-O-butyl-α-D-mannopyranoside-2,3,4-trisphosphate, 1,5-anhydro-D-arabinitol-2,3,4-trisphosphate, fructose-2,3,4-trisphosphate, 1,2-O-ethylene-β-D-fructopyranoside-2,3,4-trisphosphate, cyclohexane-1,2,3-trioltrisphosphate, 1,5-dideoxy-1,5-iminoarabinitol-2,3,4-trisphosphate, altrose-2,3,4-trisphosphate, and methyl-6-O-butyl-a-D-altropyranoside-2,3,4-trisphosphate.

6. The use according to any of claims 1 to 3 wherein the medicament is adapted for parenteral or non-parenteral administration.

7. The use according to any of claims 1 to 3 wherein the effective amount ranges from 0.1 to 100 mg per kg body weight of the mammal.

## Patentansprüche

1. Verwendung eines Monosaccharids, das ausgewählt ist aus der Gruppe, bestehend aus D/L-Ribose, D/L-Arabinose, D/L-Xylose, D/L-Lyxose, D/L-Allose, D/L-Altrose, D/L-Glucose, D/L-Mannose, D/L-Gulose, D/L-Idose, D/L-Galactose, D/L-Talose, D/L-Ribulose, D/L-Xylulose, D/L-Psicose, D/L-Sorbose, D/L-Tagatose und D/L-Fructose, worin das Monosaccharid substituiert ist mit zumindest drei vicinalen, Phosphor enthaltenden Resten der Formel: 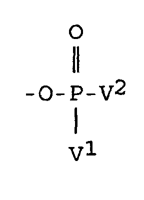 worin
V¹ und V² OH, (CH₂)ₚOH, COOH, CONH₂, CONOH, (CH₂)ₚCOOH, (CH₂)ₚCONH₂, (CH₂)ₚCONOH, (CH₂)ₚSO₃H, (CH₂)ₚSO₃NH₂, (CH₂)ₚNO₂, (CH₂)ₚPO₃H₂, O(CH₂)ₚOH, O(CH₂)ₚCOOH, O(CH₂)ₚCONH₂, O(CH₂)ₚCONOH, O(CH₂)ₚSO₃H, O(CH₂)ₚSO₃NH₂, O(CH₂)ₚNO₂, O(CH₂)ₚPO₃H₂ oder CF₂COOH sind; und
p ist 1 bis 4,
zur Herstellung eines Medikaments zur Verhinderung, Linderung oder Bekämpfung entzündlicher Zustände in Säugern, einschliesslich Menschen, oder Zuständen der Angiogenese in Säugern, einschliesslich Menschen, worin die Angiogenese mit Tumoren in Verbindung steht.

2. Verwendung gemäss Anspruch 1, worin der Tumor ein Gliom oder Prostatakrebs ist.

3. Verwendung gemäss Anspruch 1, worin der entzündliche Zustand rheumatoide Arthritis ist.

4. Verwendung gemäss irgendeinem der Ansprüche 1 bis 3, worin die Phosphor enthaltenden Reste Phosphatgruppen sind.

5. Verwendung gemäss irgendeinem der Ansprüche 1 bis 3, worin das Monosaccharid ausgewählt ist aus der Gruppe, bestehend aus Mannose-2,3,4-trisphosphat, Rhamnose-2,3,4-trisphosphat, Galactose-2,3,4-trisphosphat, Methyl-6-O-butyl-a-D-mannopyranosid-2,3,4-trisphosphat, 1,5-Anhydro-D-arabinitol-2,3,4-trisphosphat, Fructose-2,3,4-trisphosphat, 1,2-O-Ethylen-β-D-fructopyranosid-2,3,4-trisphosphat, Cyclohexan-1,2,3-triol-trisphosphat, 1,5-Didesoxy-1,5-iminoarabinitol-2,3,4-trisphosphat, Altrose-2,3,4-trisphosphat und Methyl-6-O-butyl-α-Daltropyranosid-2,3,4-trisphosphat.

6. Verwendung gemäss irgendeinem der Ansprüche 1 bis 3, worin das Medikament für die parenterale oder nichtparenterale Verabreichung angepasst ist.

7. Verwendung gemäss irgendeinem der Ansprüche 1 bis 3, worin die effektive Menge im Bereich von 0,1 bis 100 mg/kg Körpergewicht des Säugers liegt.

## Revendications

1. Utilisation d'un monosaccharide sélectionné dans le groupe du D/L-ribose, D/L-arabinose, D/L-xylose, D/L-lyxose, D/L-allose, D/L-altrose, D/L-glucose, D/L-mannose, D/L-gulose, D/L-idose, D/L-galactose, D/L-talose, D/L-ribulose, D/L-xylulose, D/L-psicose, D/L-sorbose, D/L-tagatose et D/L-fructose, ledit monosaccharide étant substitué par au moins trois radicaux vicinaux contenant du phosphore, de formule : 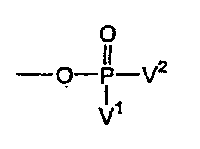 dans laquelle
V¹ et V² représentent un groupe OH, (CH₂)ₚOH, COOH, CONH₂, CONOH, (CH₂)ₚCOOH, (CH₂)ₚCONH₂, (CH₂)ₚCONOH, (CH₂)ₚSO₃H, (CH₂)ₚSO₃NH₂, (CH₂)ₚNO₂, (CH₂)ₚPO₃H₂, O(CH₂)ₚOH, O(CH₂)ₚCOOH, O(CH₂)ₚCONH₂, O(CH₂)ₚCONOH, O(CH₂)ₚSO₃H, O(CH₂)ₚSO₃NH₂, O(CH₂)ₚNO₂, O(CH₂)ₚPO₃H₂ ou CF₂COOH ; et
p est de 1 à 4,
pour la préparation d'un médicament destiné à prévenir, soulager ou combattre des conditions inflammatoires chez les mammifères, y compris l'homme, ou des conditions d'angiogenèse chez les mammifères, y compris l'homme, lorsque l'angiogenèse est liée à des conditions tumorales.

2. Utilisation selon la revendication 1, dans laquelle la condition tumorale est un gliome ou un cancer de la prostate.

3. Utilisation selon la revendication 1, dans laquelle la condition inflammatoire est l'arthrite rhumatoïde.

4. Utilisation selon l'une quelconque des revendications 1 à 3, dans laquelle les radicaux contenant du phosphore sont des groupes phosphate.

5. Utilisation selon l'une quelconque des revendications 1 à 3, dans laquelle le monosaccharide est sélectionné dans le groupe du mannose-2,3,4-triphosphate, rhamnose-2,3,4-triphosphate, galactose-2,3,4-triphosphate, méthyl-6-O-butyl-α-D-mannopyranoside-2,3,4-triphosphate, 1,5-anhydro-D-arabinitol-2,3,4-triphosphate, fructose-2,3,4-triphosphate, 1,2-O-éthylène-β-D-fructopyranoside-2,3,4-triphosphate, cyclohexane-1,2,3-triol-triphosphate, 1,5-dideoxy-1,5-iminoarabinitol-2,3,4-triphosphate, altrose-2,3,4-triphosphate et méthyl-6-O-butyl-α-D-altropyranoside-2,3,4-triphosphate.

6. Utilisation selon l'une quelconque des revendications 1 à 3, dans laquelle le médicament est adapté à une administration parentérale ou non-parentérale.

7. Utilisation selon l'une quelconque des revendications 1 à 3, dans laquelle la quantité efficace est dans la gamme de 0,1 à 100 mg par kg de poids corporel du mammifère.
